# EUROPEAN PATENT APPLICATION

(11) **EP 1 859 835 A2**
(43) Date of publication of application: **28.11.2007**
(21) Application number: 07010600.0
(22) Date of filing: 01.06.2001
(51) Int. Cl.: A61Q 19/02, A61K 8/35, A61K 8/49, A61K 8/60

(54) **Topical agent for dermatological use containing 4-hydroxyphenyl-alpha-D-glucopyranoside**

(30) Priority: 02.06.2000 JP 2000165590
(62) Divisional of application: 01945236.6
(71) Applicant: PENTAPHARM Ltd., 4002 Basel (CH); Ezaki Glico Co., Ltd., Osaka-shi, Osaka 555-8502 (JP)
(72) Inventor: Osterwalder, Uli, 4104 Oberwil (CH); Baschong, Werner, Dr., 4056 Basel (CH); Moser, Heidi, 4416 Bubendorf (CH); Nakayama, Hiroki, Tokyo (JP); Nakae, Takashi, Hyogo (JP); Kuriki, Takashi, Osaka (JP); Nishimura, Takahisa, Nara-shi, Nara (JP)
(74) Representative: Braun, André jr.

(57) **Abstract**

The objective of the present invention was to enhance the skin whitening effects and blackening prevention effects and supply safe topical agents for dermatological use. For that purpose a topical composition comprising 4-hydroxyphenyl-alpha-D-glucopyranoside and UV absorber is manufactured.

## Description

### Art to which the invention pertains

The present invention is related to topical agents for dermatological use which whiten the skin color or prevent its blackening and prevent or relieve liver spots, freckles, etc. and which show generally desirable formulation properties in terms of the safety and stability.

### Conventional technology

Various melanin formation preventing agents have been used to whiten the skin color or prevent its blackening and prevent or relieve skin troubles such as liver spots and freckles caused due to excessive exposure to UV rays. These agents include 1,4-dihydroxybenzene, β-arbutin, vitamin C and its derivatives, and kojic acid.

However, vitamin C, 1,4-dihydroxybenzene and kojic acid are extremely unstable with respect to heat and oxidation in water. When added to topical agents for dermatological use, therefore, these compounds decompose over time and cause coloration. Their derivatives, such as phosphate-ascorbyl magnesium and β-arbutin, which is obtained as a result of the β-binding of glucose to one of the hydroxy groups of 1,4-dihydroxybenzene, are not necessa-rily satisfactory in terms of efficacy although they are more stable than their parent compounds with respect to heat and oxidation.

To overcome this problem, various inventions have been made and applications for patents have been submitted, such as cosme-tics which contain, in addition to β-arbutin, ingredients with skin whitening effects, such as UV absorbents, anti-inflammatory agents, and placenta extracts, so that synergistic effects can be obtained (e.g., Toku-kai-hei 5-186324 Patent Gazette) and topical agents for dermatological use which contain pantethine-S-sulfonic acid or its salt and which prevent decomposition and coloration over time (Toku-kai-hei 5-58926 Patent Gazette). However, these substances cannot be added to cosmetic products in a quantity large enough to obtain clinically significant synergistic effects due to problems related to safety and bad feeling experienced upon contact with the skin.

The authors of the present invention looked for substances which are stable when used in topical agents for dermatological use and which are safer and more effective than conventional compounds. In this effort, they discovered 4-hydroxyphenyl-α-D-glucopyranoside as a substance which satisfies these requirements and have applied for a patent (Tokugan 2000-43366). However, its efficacy had to be further improved in order to expect clinically significant whitening effects.
A patent has been disclosed for amylase X-23 as an enzyme which transfers sugars to the phenol group of 1,4-dihydroxy-benzene through α-binding (Patent No. 2662667).

### Problems which the present invention is designed to solve

As mentioned above, various attempts have been made to enhance skin whitening effects, but none has been found satis-factory. The present invention uses 4-hydroxyphenyl-α-D-gluco-pyranoside, which shows marked skin whitening effects even when used alone, and add other substances which enhance or supplement its effects to produce topical agents with dermatological use which are more effective than conventional products.

### Methods used to solve the problems

The authors of the present invention conducted studies to solve the above-mentioned problems, found that the combination of 4-hydroxyphenyl-α-D-glucopyranoside and specific ingredients (hereafter sometimes referred to as "auxiliary ingredients") results in a marked enhancement of the effects of 4-hydroxyphe-nyl-α-D-glucopyranoside, which shows far greater skin whitening effects than conventional products even when used alone, and completed the present invention.

The present invention pertains to topical agents for derma-tological use which are characterized by the fact that they con-tain 4-hydroxyphenyl-α-D-glucopyranoside and at least one auxiliary ingredient like e.g. one of the following: ascorbic acid and its derivatives, crude drugs and their extracts, hydroxycarboxylic acid and its salts, oil-soluble glycyrrhiza extract, gentian extract, phenol derivatives and its salts, placenta extract, kojic acid and its derivatives, glucos-amine and its derivatives, azelaic acid and its derivatives, re-tinol and its derivatives, pyridoxin and its derivatives, toco-pherol and its derivatives, vitamin E-nicotinate, diisopropyl-amine-dichloroacetate, chitosan and its decomposition products, caffeic acid derivatives, hydroxycinnamate and its derivatives, Umbelliferae plant extracts, mycelial cultures and their extracts, plants leaves and their extracts, plant bark and its extracts, hinokitiol, ginseng extract, sulfur, crude sugar extracts, molasses extracts, mucopolysaccharide, teprenone, nordihydroguaiaretic acid, UV absorbents, γ-pyrone glycoside, hydroxysalicylic acid glycoside, hydroxysalicylic acid fatty ester glycoside, biphenyl compounds, ceramides, substances with ceramide-like structures, ether compounds which can be shown by the general formula R31-O-(X-O)n-R32 (in which R31 and R32 are the same or different normal-chain, branched or cyclic alkyl groups with 1 to 12 carbon atoms, X is alkylene groups with 1 to 12 carbon atoms, n is 0 or 1, and the number of synthetic carbon atoms in R31, R32 and X is 10 to 32), pantothenic acid and its derivatives, sodium hydrogen sulfite, antiinflammatory agents, allantoin and its derivatives, amino acid and its derivatives, amino ethyl compounds, alkylene diamine carboxylic acid deriva-tives, betaine derivatives, acyl methyl taurine, fibronectin, tyrosinase inhibitors, hederacoside and its salts, gymnema saponin, beat saponin and its salts, ellagic acid-related compounds and their alkaline metallic salts, and resorcinol derivatives. It also pertains to topical agents for dermato-logical use described above which are characterized by the fact that 4-hydroxyphenyl-α-D-glucopyranoside is obtained using α-amylase, as well as those in which α-amylase is amylase X-23.

### Working of the invention

The auxiliary agents under the present invention, when at least one of them is added to topical agents for dermatological use in combination 4-hydroxyphenyl-α-D-glucopyranoside, increase whitening effects or stability of 4-hydroxyphenyl-α-D-glucopyra-noside. Explanations of these auxiliary agents are provided below.

Among various types of ascorbic acid, L-ascorbic acid, generally called vitamin C, promotes cell respiration, enzymatic activation, and collagen formation due to its strong reducing effects and also reduces melanin. Derivatives of ascorbic acid include ascorbate monoalkylesters (such as ascorbate monostea-rate, ascorbate monopalmitate and ascorbate monooleate), ascor-bate monoester derivatives (such as ascorbate monophosphate ester and ascorbate-2-sulfate), ascorbate diester derivatives (such as ascorbate distearate, ascorbate dipalmitate, ascorbate dioleate and ascorbate diphosphate esters), ascorbate trialkyl esters (such as ascorbate tristearate, ascorbate tripalmitate and ascorbate trioleate), and ascorbate triester derivatives (such as ascorbate triphosphate ester).
Efficacy is seen when these ingredients are added at 0.01 w/w% or more in topical agents for dermatological use. The upper limit of content is about 10%.

Usable crude drugs include the following and their extracts can also be used: mulberry bark, peony root, scutellaria root, chamomile, Japanese angelica root, rosemary, geranium herb, lithospermum root, tea leaf, pueraria root, clove, glycyrrhiza, biwa, bitter orange peel, ginseng, sanzasi, ophiopogon tuber, ginger, pine cone, magnolia bark, gambir, aloe, marshmallow, meadow sweet, water cress, cinchona, comfrey, scopolia rhizome, swertia herb and yarrow (Achillea millefolium Linn'e) (Composi-tae). Under the present invention, crude drugs and their ex-tracts include fine power obtained by pulverizing (and drying, if necessary) the whole plants, roots, leaves, flowers, seeds, etc. of the above mentioned crude drugs, extracts obtained by soaking these materials in water and/or organic solvents and filtering the residue, fluid obtained by removing the solvent from these extracts, and these power products or extracts with or without the solvents dissolved, dispersed, or diluted with appropriate solvents or dissolving agents.
The content of these materials in topical agents for dermatological use should be 0.001-20 w/w%, preferably 0.01-10 w/w%.

Hydroxycarboxylic acids include glycollic acid, lactic acid, malic acid, tartaric acid, citric acid, salicylic acid, mevalonic acid, and lactone mevalonate. Their salts include metallic salts such as Na, K and Mg, as well as organic salts such as trietha-nolamine and 2-amino-2-methyl-1,3-propandiol.
The content of these ingredients in topical agents for dermatological use should be 0.0001-5 w/w%, preferably 0.001-3 w/w%.

Oil-soluble glycyrrhiza extracts are obtained by extracting glycyrrhiza (Glycyrrhizaglabra linne), a perennial legume, with lower monohydric alcohol such as methyl alcohol and ethyl alcohol and fluid polyhydric alcohol such as glycerin, propylene glycol, and 1,3-butylene glycol. Any preparation method may be used, e.g., extraction using various appropriate solvents at low temperature or room temperature or with heating. Preferably, extrac-tion should be conducted as follows: extract with ethyl alcohol for 2-10 hours while heating; filter; allow the obtained filtrate to stand for 2 to 3 days and allow it to mature; and filter again. The extract obtained may be condensed and dried, if necessary, after extraction with heating. Oil-soluble glycyrrhi-za extracts thus obtained are a brown substance with a peculiar odor. They can be used as is in many cases but may be deodorized or decolorized to purify them, if necessary, as long as their efficacy is not compromised. Purification may be conducted using, for example, an active carbon column. Any method commonly applied to extracts may be used for purification.
The content may vary between 0.0001 and 5% w/w%, preferably between 0.001 and 3% w/w%, depending on the quality of the extract used and other factors.

Gentian extracts can be obtained by extracting the root and rhizome of gentian (Gentiana litea linne (Gentianaceae)), a plant which belongs to the Gentianaceae family, with lower monohydric alcohol such as methyl alcohol and ethyl alcohol and fluid poly-hydric alcohol such as glycerin, propylene glycol, and 1,3-butylene glycol. Any preparation method may be used, e.g., extraction using various appropriate solvents at low temperature or room temperature or with heating. Preferably, extraction should be conducted as follows: extract with 50% 1,3-butylene glycol in water for 2-10 hours while heating; filter; allow the obtained filtrate to stand for 2 to 3 days and allow it to mature; and filter again. The extract obtained may be condensed and dried, if necessary, after extraction with heating.
The content may vary between 0.0001 and 5% w/w%, preferably between 0.001 and 3% w/w%, depending on the quality of the extract used and other factors.

Phenol derivatives and its salts include 4-ethoxyphenol, 4-n-propoxyphenol, 4-n-butoxyphenol, 4-n-hexadecyloxyphenol, 4-n-octadecyloxyphenol, 4-ethylphenol, 4-n-propylphenol, 4-n-butyl-phenol, 4-t-butylphenol, 4-isopropylphenol, 4-hexadecylphenol, 4-octadecylphenol, 4-isopropylcatecholmonocutylester, and 4-isopropylcatecholmonoheptadecaester.
The content may vary between 0.01 and 20% w/w%, preferably between 0. 1 and 10% w/w%, in topical agents for dermatological use.

Placenta extracts include extracts obtained by soaking the placenta from humans, monkeys, cows, pigs, sheep, mice and other animals in water and/or organic solvents and filtering the resi-due, fluid obtained by removing the solvent from these extracts, and power of these placenta or the above-mentioned extracts with or without the solvents dissolved, dispersed, or diluted. Specifically, these placenta extracts are commercially available as water or oil-soluble placenta extracts.
The content in topical agents for dermatological use should be 0.001-5 w/w%, preferably 0.1-3 w/w%.

Kojic acid and its derivatives include monoesters such as kojic acid, kojic acid glycoside, kojic acid monobutyrate, kojic acid monocaprate, kojic acid monopalmitate, kojic acid monostea-rate, kojic acid monocinnamate, and kojic acid monobenzoate, as well as diesters such as kojic acid dibutyrate, kojic acid dipalmitate, kojic acid distearate, and kojic acid dioleate.
The content in topical agents for dermatological use should be 0.001-30 w/w%, preferably 0.01-10 w/w%, and more preferably 0.01-5 w/w%.

Glucosamine and its derivatives include glucosamine, glucosamine-6-phosphate, and glucosamine-6-sulfate.
The content in topical agents for dermatological use should be 0.001-5 w/w%, preferably 0.1-3 w/w%.

Azelaic acid and its derivatives include azelain and azelaic acid.
The content in topical agents for dermatological use should be 0.001-5 w/w%, preferably 0.1-3 w/w%.

Retinol is generally called vitamin A1 and is effective in maintaining normal function of the skin and mucosa. Its derivatives include retinal and retinoic acid.
The content in topical agents for dermatological use should be 0.001-5 w/w%, preferably 0.1-3 w/w%.

Pyridoxin is a substance with vitamin B6 effects, and its derivatives include pyridoxal, pyridoxamine, pyridoxin-5'-phosphate, pyridoxal-5'-phosphate, pyridoxamine-5'-phosphate, pyridoxal phosphate, and pyridoxinic acid.
The content in topical agents for dermatological use should be 0.001-5 w/w%, preferably 0.1-3 w/w%.

Tocopherol, a group of vitamin E derivative, is effective in preventing and treating hyperkeratosis and other diseases and preventing and reversing aging of the skin. This group includes α-tocopherol, β-tocopherol, γ-tocopherol and β-tocopherol. Their derivatives may also be used in the present invention
The content in topical agents for dermatological use should be 0.001-5 w/w%, preferably 0.1-3 w/w%.

Alpha-tocopherol derivatives include α-tocopheryl retinoate, which is vitamin A acid ester. Here, α-tocopherol refers to DL-α-tocopherol, D-α-tocopherol or mixed natural tocopherol containing D-α-tocopherol. Vitamin A acid refers to retinoic acid (all-trans-retinoic acid), 13-cis-retinoic acid, 11-cis-retinoic acid, 9-cis-retinoic acid or their mixed isomers. Ester of DL-α-tocopherol and all-trans-retinoic acid is particularly preferable.

Vitamin E-nicotinate and diisopropylamine dichloroacetate improve blood flow, activate cells, inhibit the formation of melanin due to UV rays, promote the elimination of melanin, prevent epidermal drying, accelerate skin metabolism, and prevent aging of the skin due to UV rays.
The content of vitamin E-nicotinate or diisopropylamine dichloroacetate in topical agents for dermatological use should be 0.01-5 w/w%.

Chitosan is produced as a result of deacetylation of chitin and has a β-1,4-polyglucosamine structure. Decomposition pro-ducts of chitosan are obtained as a result of treatment of chito-san with enzymes such as chitinase and contain glucosamine and its oligomers.
The content in topical agents for dermatological use should be 0.001-5 w/w%, preferably 0.1-3 w/w%.

The content of caffeic acid derivatives in topical agents for dermatological use should be 0.001-5 w/w%, preferably 0.1-3 w/w%.

Hydroxycinnamate and its derivatives include hydroxycinnamic acid (including p-coumarinic acid and p-coumaric acid) and coffeic acid.
The content in topical agents for dermatological use should be 0.001-5 w/w%, preferably 0.1-3 w/w%.

Umbelliferae plant extracts include extracts obtained by soaking the whole plants, roots, leaves, flowers, seeds, etc. of umbelliferae plants (such as ledebouriella, glehnia root, Noto-pterygium incisium Ting, cnidium rhizome, angelica dahurica root, Ligusticum sinense Oliv., dokkatu, zenko and bupleurum) in water and/or organic solvents and filtering the residue, fluid obtained by removing the solvent from these extracts, and these power pro-ducts or extracts with or without the solvents dissolved, dispersed, or diluted with appropriate solvents or dissolving agents.
The content in topical agents for dermatological use should be 0.001-5 w/w%, preferably 0.1-3 w/w%.

Mycelial cultures refer to mycelia of mushroom and reisi cultured on appropriate media and include culture fluid itself in the case of liquid culture and mycelia pulverized after drying, etc., if necessary, in the case of solid culture. Extracts of mycelial cultures include extracts obtained by soaking the above-mentioned mycelial cultures, mycelia or their powder in water and/or organic solvents and filtering the residue, fluid obtained by removing the solvent from these extracts, and these power pro-ducts or extracts with or without the solvents dissolved, disper-sed, or diluted with appropriate solvents or dissolving agents.
The content in topical agents for dermatological use should be 0.001-5 w/w%, preferably 0.1-3 w/w%.

Plant leaves include leaves from plants such as apple, Japanese pieris, amasiba (Japanese name), and gymnema. Leaves are pulverized after drying, if necessary. Extracts of plant leaves include extracts obtained by soaking these leaves or their powder in water and/or organic solvents and filtering the resi-due, fluid obtained by removing the solvent from these extracts, and these power products or extracts with or without the solvents dissolved, dispersed, or diluted with appropriate solvents or dissolving agents.
The content in topical agents for dermatological use should be 0.001-20 w/w%, preferably 0.1-3 w/w%.

Plant bark includes the bark from trees of fruits such as apple, cherry, peach, and pear. The bark is pulverized after drying, if necessary. Extracts of the bark include extracts ob-tained by soaking the bark or its powder in water and/or organic solvents and filtering the residue, fluid obtained by removing the solvent from these extracts, and these power products or extracts with or without the solvents dissolved, dispersed, or diluted with appropriate solvents or dissolving agents.
The content in topical agents for dermatological use should be 0.001-5 w/w%, preferably 0.1-3 w/w%.

The content of hinokitiol in topical agents for dermatologi-cal use should be 0.001-5 w/w%, preferably 0.1-3 w/w%.

Ginseng extracts include extracts obtained by soaking gin-seng or its powder in water and/or organic solvents and filtering the residue, fluid obtained by removing the solvent from these extracts, and these power products or extracts with or without the solvents dissolved, dispersed, or diluted with appropriate solvents or dissolving agents. These extracts are commercially available.
The content in topical agents for dermatological use should be 0.001-5 w/w%, preferably 0.1-3 w/w%.

The content of sulfur in topical agents for dermatological use should be 0.001-5 w/w%, preferably 0.1-3 w/w%.

Crude sugar extracts are brown pigment ingredients. Dried power is hygroscopic and has a slight burning odor and a slight bitter taste. Their production methods are described in Toku-Kai-Sho No. 60-78912 Patent Gazette. Specifically, crude sugar (black sugar) or molasses (byproduct obtained in the production of white sugar from black sugar) is dissolved in an appropriate amount of water, and pigment ingredients are adsorbed by bringing them into contact with adsorbents such as non-polar polystyrene resin adsorbents. Adsorbents are washed with water to thoroughly eliminate sugar. The pigment ingredients adsorbed to the adsor-bents are eluted with hydrous alcohol of 20% or higher concentra-tions. After condensation or freeze drying, the pigment ingre-dients are refined by recrystallization, if necessary, by evapo-rating the ingredients to dryness.
The content in topical agents for dermatological use should be 0.01-10 w/w%, preferably 0.1-5 w/w%.

Molasse extract's main ingredient is oligosaccharide and can be obtained by soaking molasses in cold or warm lower alcohol such as methanol and ethanol and filtering, condensing and discoloring the fluid obtained.
The content in topical agents for dermatological use should be 0.01-10 w/w%, preferably 0.1-5 w/w%.

Mucopolysaccharide shows skin moisturizing effects and includes hyaluronic acid, chondroitin-4-sulfate, chondroitin-6-sulfate, dermatan sulfate, heparin and their salts.
The content in topical agents for dermatological use should be 0.001-10 w/w%, preferably 0.01-5 w/w%.

Teprenone, chemically named geranyl geranyl acetone, pro-tects the mucosa and promotes its repair, activates cell growth, and accelerates the synthesis of phospholipid. Teprenone was also found to inhibit tyrosinase, an enzyme involved in the biosyn-thesis of melanin which causes liver spots, freckles, and black skin (Toku-Kai-Hei No. 6-16532 Patent Gazette).
The content in topical agents for dermatological use should be 0.01-20 w/w%, preferably 0.5-10 w/w% and more preferably 1.0-10 w/w%.

Nordihydroguaiaretic acid, generally known as an antioxidant and a lipoxygenase inhibitor, is added to cosmetic and pharmaceu-tical products to prevent oxidation and stabilize formulations.
The content in topical agents for dermatological use should be 0.001-10 w/w%, preferably 0.1-5 w/w%.

Any UV absorbents commonly used in topical agents for dermatological use can be used as UV adsorbents in the present invention. Typical UV adsorbents are listed below.

1) Benzoate UV absorbents
   Paraaminobenzoic acid (PABA), PABA monoglycerin ester, N,N-bis-(3-hydroxypropyl) PABA ethyl ester, N,N-bis-(2-hydroxyethyl) PABA ethyl ester, N,N-dimethyl PABA ethyl ester, N,N-dimethyl PABA butylester, N,N-dimethyl PABA amylester, and N,N-dimethyl PABA octyl ester.

2) Anthranilate UV absorbents
   Homomenthyl-N-acetylanthranilate

3) Salicylate UV absorbents
   Amylsalicylate, methylsalicylate, homomenthylsalicylate, octylsalicylate, phenylsalicylate, benzylsalicylate, and 4-isopropylphenylsalicylate

4) Cinnamate UV absorbents
   Octylcinnamate, ethyl-4-isopropylcinnamate, methyl-2,5-diisopropylcinnamate, ethyl-2,4-diisopropylcinnamate, methyl-2,4-diisopropylcinnamate, propyl-4-methoxycinnamate, isopropyl-4-methoxycinnamate, isoamyl-4-methoxycinnamate, isopropyl-4-methoxycinnamate, isoamyl-4-methoxycinnamate, octyl-4-methoxy-cinnamate (2-ethylhexyl-4-methoxycinnamate), 2-ethoxyethyl-4-methoxycinnamate, cyclohexyl-4-methoxycinnamate, ethyl-α-cyano-β-phenylcinnamate, 2-ethylhexyl-α-cyano-β-phenylcinnamate, and glyceryl mono-2-ethylhexanoyl-bis-(paramethoxycinnamate)

5) Benzophenone UV absorbents
   2,4-dihydroxybenzophenone, 2,2'-dihydroxy-4-methoxybenzo-phenone, 2,2'-dihydroxy-4,4'-dimethoxybenzophenone, 2,2'-4,4'-tetrahydroxybenzophenone, 2-hydroxy-4-methoxy-benzophenone, 2-hydroxy-4-methoxy 4'-methylbenzophenone, 2-hydroxy-4-methoxy-benzophenone-5-sulfonate, 4-phenylbenzophenone, 2-ethylhexyl-4'-phenylbenzophenone-2-carboxylate, 2-hydroxy-4-n-octoxybenzo-phenone, and 4-hydroxy-3-carboxybenzophenone.

6) Other UV absorbents
   3-(4'-methylbenzylidene)-d,1-camphor, 3-benzylidened,1-camphor, urocanic acid, ethyl urocanate, 2-phenyl-5-methylbenz-oxazol, 2-(2'-hydroxy-5'-ethylphenyl)-benzotriazol, 2-(2'-hydroxy-5'-t-butylphenyl)-benzotriazole, 2-(2'-hydroxy-5'-methylphenyl)-benzotriazole, dibenzalazine, dianisoylmethane, 4-methoxy-4'-t-butyldibenzoylmethane, 5-(3,3-dimethyl-2-norbornylidene)-3-pentane-2-one.

The content in topical agents for dermatological use should be 0.01-10 w/w%, preferably 0.5-8 w/w%. If the content is too small, sunburn can not be prevented and the whitening effects of 4-hydroxyphenyl-α-D-glucopyranoside are reduced. If the content is too large, stability of topical agents for dermatological use is affected.

γ-Pyrone glycoside prevents liver spots and freckles due to sunburn. It is maltol-3-O-(6'-O-apiocil)-glucoside or maltol-3-O-glucoside which can be shown by general formula 1 shown below. For example, it can be collected by column chromatography, HPLC, TLC, etc. from pueraria root extracts.
The content in topical agents for dermatological use should be 0.00001-2.5 w/w%, preferably 0.0001-1 w/w%.

Chemistry 1 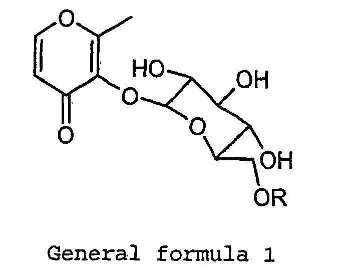 In the above formula, R is a hydrogen atom or the group shown below.

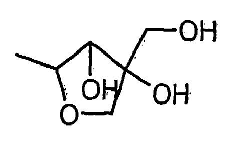

Hydroxysalicylic acid glycoside and hydroxysalicylic acid fatty ester glycoside show excellent whitening effects as a result of synergism with 4-hydroxyphenyl-α-D-glucopyranoside and can be shown by general formulas 2, 3 and 4.
These glycosides can be obtained by allowing hydroxysalicy-lic acid or hydroxysalicylic acid fatty ester to react with ace-tylated sugar such as pentaacetylglucose (or aceto-bromated sugar such as acetobromoglucose) in the presence of acidic catalysts.
The content in topical agents for dermatological use should be 0.001-20 w/w%, preferably 0.1-7 w/w%.

Chemistry 2

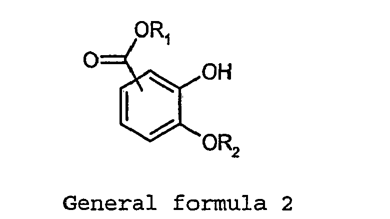

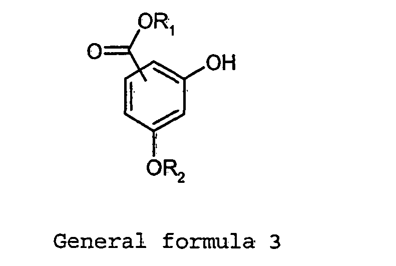

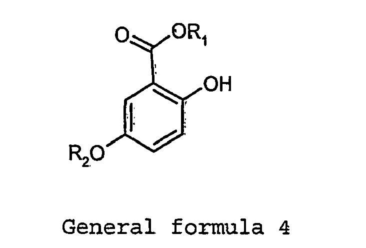

In general formulas 2 through 4, R1 is a hydrogen atom or saturated or unsaturated normal-chain or branched hydrocarbon group with 1 to 20 carbon atoms, while R2 is a sugar residue.

Examples of the above-mentioned glycosides are listed below.
3-β-D-glucopyranosyloxy salicylic acid, 3-β-D-glucopyranosyloxy methyl salicylate, 3-β-D-glucopyranosyloxy ethyl salicylate, 3-β-D-glucopyranosyloxy propyl salicylate, 3-β-D-glucopyranosyloxy isopropyl
salicylate, 4-β-D-glucopyra-nosyloxy salicylic acid, 4-β-D-glucopyranosyloxy methyl salicylate, 4-β-D-glucopyranosyloxy ethyl salicylate, 4-β-D-glucopyranosyloxy propyl salicylate, 4-β-D-glucopyrano-syloxy isopropyl salicylate, 5-β-D-glucopyranosyloxy salicylic acid, 5-P-D-glucopyranosyloxy methyl salicylate, 5-β-D-glucopyranosyloxy ethyl salicylate, 5-β-D-glucopyranosyloxy propyl salicylate, 5-β-D-glucopyranosyloxy isopropyl salicylate, 6-β-D-glucopyranosyloxy salicylic acid, 6-β-D-glucopyranosyloxy methyl salicylate, 6-β-D-glucopyranosyloxy ethyl salicylate, 6-β-D-glucopyranosyloxy propyl salicylate, 6-β-D-glucopyranosyloxy isopropyl salicylate, 2-β-D-glucopyrano-syloxy-3-hydroxybenzoic acid, 2-β-D-glucopyranosyloxy-3-methyl hydroxybenzoate, 2-β-D-glucopyranosyloxy-3-ethyl hydroxybenzo-ate, 2-β-D-glucopyranosyloxy-3-propyl hydroxybenzoate, 2-β-D-glucopyranosyloxy-3-isopropyl hydroxybenzoate, 2-β-D-glucopyra-nosyloxy-4-hydroxybenzoic acid, 2-β-D-glucopyranosyloxy-4-methyl hydroxybenzoate, 2-β-D-glucopyranosyloxy-4-ethyl hydroxybenzo-ate, 2-β-D-glucopyranosyloxy-4-propyl hydroxybenzoate, 2-β-D-glucopyranosyloxy-4-isopropyl hydroxybenzoate, 2-β-D-glucopyra-nosyloxy-5-hydroxybenzoic acid, 2-β-D-glucopyranosyloxy-5-methyl hydroxybenzoate, 2-β-D-glucopyranosyloxy-5-ethyl hydroxybenzo-ate, 2-β-D-glucopyranosyloxy-5-propyl hydroxybenzoate, and 2-β-D-glucopyranosyloxy-5-isopropyl hydroxybenzoate

Biphenyl compounds inhibit tyrosinase activity and melanin formation and can be shown by general formulas 5 and 6.
Specifically, biphenyl compounds include dehydrocreosol, dehydrodieugenol, and tetrahydromagnolol.
The content in topical agents for dermatological use should be 0.0001-20 w/w%, preferably 0.001-5 w/w%.

Chemistry 3

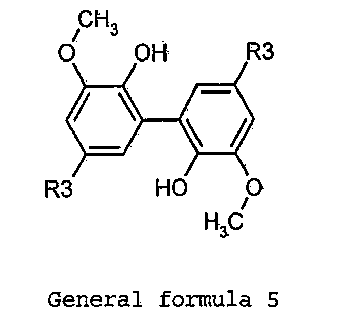

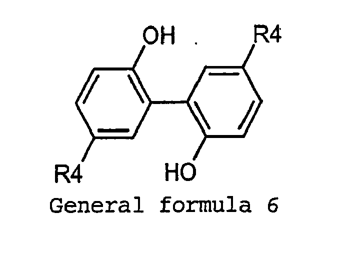

In general formulas 5 and 6, R3 is CH₃, C₂H₅, C₃H₇, CH₂OH, C₃H₆OH, CH₂CH=CH₂, while R4 is a hydrogen atom or saturated normal-chain or branched hydrocarbon group with 1 to 8 carbon atoms.

Ceramides and substances with ceramide-like structures moisten, soften and whiten the skin, alleviate inflammation, antagonize oxidation, and promote blood flow. Ceramides are shown by general formula 7, while substances with ceramide-like structures are shown by general formulas 8, 9, 10, 11, and 12.
Ceramides and substances with ceramide-like structures can be used in combination (combination of 1 or more of the ceramides and/or substances with ceramide-like structures). The content in topical agents for dermatological use should be 0.01-50 w/w%, preferably 0.01-20 w/w% and more preferably 0.1-10 w/w%. These substances show moisturizing effects and prevent and relieve rough skin with good stability and feeling upon contact with the skin.

Chemistry 4

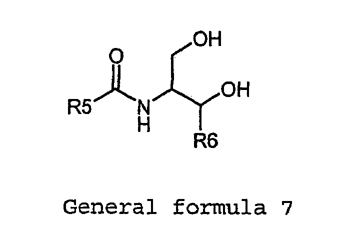

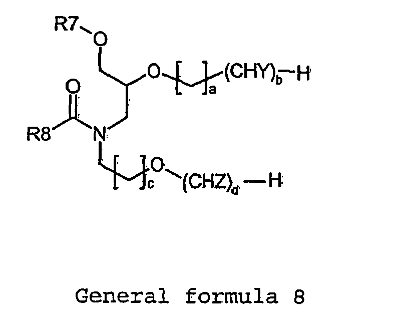

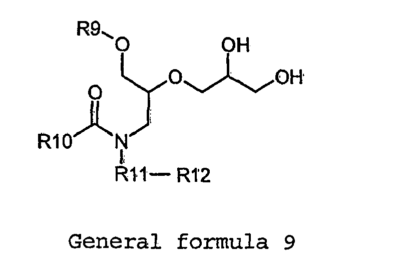

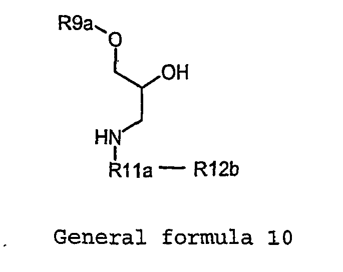

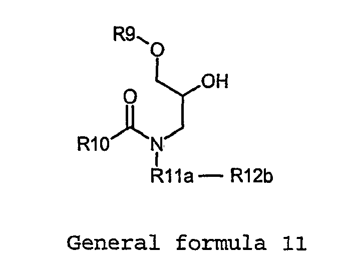

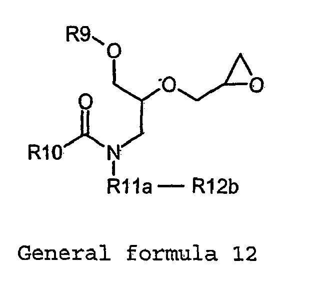

In general formula 7, R5 and R6 are the same or different hydroxyl group-substituted normal-chain or branched, saturated or unsaturated hydrocarbon groups with 8 to 26 carbon atoms.
In general formula 8, R7 is a normal-chain or branched, saturated or unsaturated hydrocarbon group with 10 to 26 carbon atoms; R8 is a normal-chain or branched, saturated or unsaturated hydrocarbon group with 9 to 25 carbon atoms; Y and Z are a hydrogen atom or a hydroxyl group; a is 0 or 1; c is an integral number of 0 to 4; and b and d are integral numbers of 0 to 3.
In general formula 9, R9 and R10 are the same or different normal-chain or branched, saturated or unsaturated, hydroxylated or non-hydroxylated hydrocarbon groups with 1 to 40 carbon atoms; R11 is a normal-chain or branched alkylene group with 1 to 6 carbon atoms or a single bond; and R12 is a hydrogen atom, normal-chain or branched alkoxy group with 1 to 12 carbon atoms or 2,3-dihydroxypropyloxy group. When R11 is a single bond, R12 is a hydrogen atom.
In general formula 10, R9a is a hydroxylated or nonhydroxy-lated hydrocarbon radical with 4 to 40 carbon atoms; R11a is a normal-chain or branched alkylene group with 3 to 6 carbon atoms; and R12a is a normal-chain or branched alkoxy group with 1 to 12 carbon atoms.
In general formula 11, R9, R10, R10a, and R12a are the same as above.
In general formula 12, R9, R10, and R11 are the same as above; and R12b is a hydrogen atom or a normal-chain or branched alkoxy group with 1 to 12 carbon atoms or 2,3-dihydroxypropyloxy group. When R11 is a single bond, R12b is a hydrogen atom.

Ether compounds which can be shown by the general formula R21-O-(X-O)n-R22 increase percutaneous absorption of the topical agents for dermatological use pertaining to the present invention without irritating the skin.
In this general formula, R21 and R22 may be the same or dif-ferent and are normal-chain, branched or cyclic alkyl groups with 1 to 12, preferably 2 to 22 and more preferably 3 to 20, carbon atoms. It is preferable that R21 and/or R22 are branched at 2 or more sites, preferably at 2 sites. Specifically, these groups in-clude the following: methyl group, butyl group, n-butyl group, n-decyl group, n-dodecyl group, n-tetradecyl group, n-octadecyl group, n-eicosyl group, n-tetracosyl group, 1-methylpropyl group, 3-methylhexyl group, 2-methylheptadecyl group, 1,3-dimethylbutyl group, 1,3-dimethylpentyl group, and cyclopentyl group.
X is an alkylene group with 1 to 12, preferably 1 to 8, car-bon atoms, specifically, methylene group, ethylene group, butyle-ne group, etc.
The combined number of carbon atoms in R21 and R22 must be 10 to 32, preferably 12 to 28.
n is 0 or 1, preferably 0.

These ether compounds can be manufactured by known methods, e.g., direct etherification of corresponding alcohol and alkyl halide, reduction of aryl ether obtained as a result of addition of corresponding alcohol and olefin in the presence of Lewis acid catalyst or as a result of addition of corresponding alcohol and alkyl halide in the presence of alkaline catalyst, and reduction of acetal or ketal produced from corresponding alcohol and aldehyde or ketone.
The content in topical agents for dermatological use should be 0.01-50 w/w%, preferably 0.01-20 w/w% and more preferably 0.1-10 w/w%.

Pantothenic acid is a vitamin B. It not only shows skin whi-tening effects but increases stability of 4-hydroxyphenyl-α-D-glucopyranoside in topical agents for dermatological use. Ist de-rivatives include pantethine-S-sulfonic acid, 4'-phosphopantheti-ne-S-sulfonic acid, pantethine, and glucopyranosyl pantothenate. These compounds can be used in the form of not only free acid but salt as well. Salts include a wide range of organic and inorga-nic acid salts, but alkaline metal salts and alkali earth metal salts, e.g. calcium d-pantetheine-s-sulfonate are preferable.
The content in topical agents for dermatological use should be 0.001-5 w/w%, preferably 0.1-3 w/w%. The content should be ad-justed so that the ratio of weight of 4-hydroxyphenyl-α-D-gluco-pyranoside and pantothenic acid and/or its derivatives is not less than 1:0.1, preferably 1:0.1 to 10 and more preferably 1:0.5 to 5.

Sodium hydrogen sulfite is known to increase the stability of β-arbutin in topical agents for dermatological use (Patent No. 2.107858). Sodium hydrogen sulfite also increase the stability of 4-hydroxyphenyl-α-D-glucopyranoside in topical agents for dermatological use.
The content should be adjusted so that 4-hydroxyphenyl-α-D-glucopyranoside:sodium hydrogen sulfite ratio (weight) is 1:0.0001 to 1, preferably 1:0.001 to 0.1.

Anti-inflammatory agents are used to prevent inflammation and other adverse reactions that may be caused by some of the auxiliary agents used in the present invention. Any anti-inflam-matory agents applicable to the skin can be used, such as oxyben-zone, tranexamic acid and its derivatives, ε-aminocaproic acid, glycyrrhizic acid, azulene, sensitizing agent No. 301, sensiti-zing agent No. 401, diphenhydramine HCl, adenosine phosphate, calamine, lithospermum root extract, mugwort extract, sarguisorba extract, aminocaproic acid and bisabolol.
The content in topical agents for dermatological use should be 0.01-2 w/w%, preferably 0.1-2 w/w%.

Allantoin is used to treat various dermatological diseases and is effective in the treatment of skin wound and prevention of rough skin. Its derivatives include dihydroxy aluminum allantoi-nate and chlorohydroxy aluminum allantoinate.
The content in topical agents for dermatological use should be 0.01-5 w/w%, preferably 0.1-3 w/w%.

Amino acid is used to rehydrate aged or hardened epidermis. Neutral amino acid (such as glycine, serine, cystine, alanine, threonine, cysteine, valine, phenylalanine, methionine, leucine, tyrosine, proline, isoleucine and hydroxyproline), acidic amino acid (such as aspartic acid, asparagine, glutamine and glutamic acid), and basic amino acid (arginine, histidine and lysine) can be used. Amino acid derivatives include acylsarcosine and its salts, acylglutamic acid and its salts, acyl-β-alanine and its salts, glutathione, and pyrrolidone carboxylic acid and its salts, as well as oligopeptides such as glutasin, carnosine, gramicidin S, tyrocidin A and tyrocidin B, γ-aminobutyric acid and γ-amino-β-hydroxybutyric acid and its salts.
The content in topical agents for dermatological use should be 0.01-20 w/w%, preferably 0.05-10 w/w%. The skin moisturizing effects may be insufficient if the content is too small. If the content is too large, alteration of amino acid becomes difficult to prevent without increases in its beneficial effects.

Amino ethyl compounds, shown by the formula NH₂CH₂CH₂X (in which X is -SO₂H or -SO₂SH), are used to prevent and relieve rough skin and relieve subduedness.
The content in topical agents for dermatological use should be 0.0001-1.0 w/w%, preferably 0.001-0.3. w/w%.

Alkylene diamine carboxylic acid derivatives are used to increase stability of topical agents for dermatological use. Ethylene diamine tetraacetate and its alkali metal salts (such as Na, K and Li salts), alkali earth metal salts (such as Ca and Mg), ammonium salt, and alkanol salts are preferable, but Na salt is most preferable.
The content in topical agents for dermatological use should be 0.01-0.5 w/w%, preferably 0.05-0.5 w/w%.

Betaine derivatives are used to increase percutaneous ab-sorption of 4-hydroxyphenyl-α-D-glucopyranoside, and alkyl dime-thyl amino acid showed by general formula 13, 2-alkyl-1-carboxy-methyl-1-hydroxyethyl-2-imidazoline shown by general formula 14, N-(3-acylaminopropyl)-N,N-dimethylamino acetate shown by general formula 15, and N-alkyl-N,N-dimethyl-3-amino-2-hydroxypropane sulfonic acid shown by general formula 16 are desirable.

Acyl methyl taurine is also used to increase percutaneous absorption of 4-hydroxyphenyl-α-D-glucopyranoside and can be shown by general formula 17.

Chemistry 5

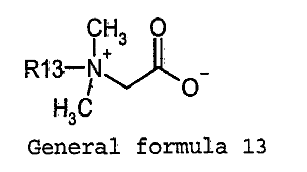

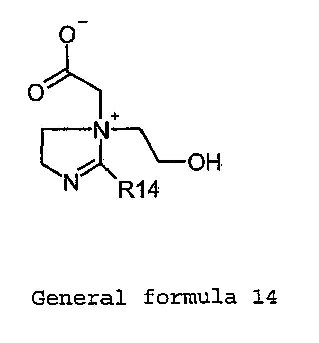

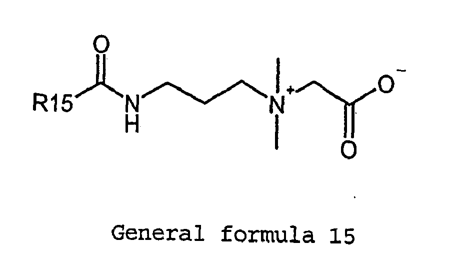

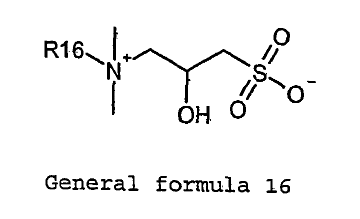

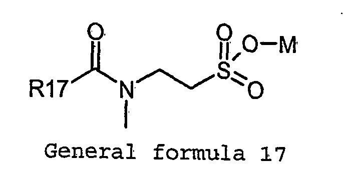

In general formulas 13, 14, 15, 16 and 17, R13 and R16 are normal-chain or branched alkyl groups with 8 to 24 carbon atoms; R14, R15 and R17 are normal-chain or branched alkyl groups with 7 to 23 carbon atoms; M is a univalent or bivalent metal, ammonium, alkanol amine or hydrogen atom.

The combined content of betaine derivatives and acyl methyl taurine in topical agents for dermatological use should be 0.01-30 w/w%, preferably 0.1-20 w/w%.

Fibronectin (cold insoluble globulin) increases whitening effects of 4-hydroxyphenyl-α-D-glucopyranoside used in the present invention.
The suitable content in topical agents for dermatological is 0.000001-0.1 w/w%.

Tyrosinase inhibitors are added to enhance the inhibition of tyrosinase activity by 4-hydroxyphenyl-α-D-glucopyranoside or to give greater anti-tyrosinase effects to the topical agents for dermatological use covered by the resent invention by way of synergism with 4-hydroxyphenyl-α-D-glucopyranoside. To obtain tyrosinase inhibitors, cells of *Catharanthus roseus* L. (group of cells or tissue strips such as the root, embryonic axis, and cotyledon of young plants and the root, stem, petiole, flower, and pollen of mature plants) are cultured on medium to which plant growth controlling agents containing plant hormones such as auxin and cytokinin are added in order to induce callus, or tumor tissues are produced using agrobacterium tumefaciens or agrobac-terium rhizogenes. The callus or tumor tissue is cultured using 4-hydroxyphenyl-α-D-glucopyranoside containing media (such as Murashige-Skoog medium, Linsmaier-Skoog medium, White medium, Gamborg medium, Nitsch medium, Heller medium, Schenk-Hildebrandt medium, Nitzsch-Nitzsch medium, and Kohlenbach-Schmidt medium) and the cultures obtained are homogenized. The transparent fluid deriving from the homogenate obtained is used per se or after drying as a tyrosinase inhibitor.
The content should be adjusted so that appropriate antityrosinase activity is obtained.

Hederacoside enhances the whitening effects of the topical agents for dermatological use covered by the present patent as a result of synergism with 4-hydroxyphenyl-α-D-glucopyranoside. Hederacoside is a triterpenoid saponin obtained from extracts of Sapindus *mukurossi Gaertn.* or Akebia quinata Decne. Its salts include alkali metal salts such as Na and K salts, ammonium salt, basic amino acid salts, alkanol amine salts, and esters. These extracts can be used as is.
The content in topical agents for dermatological use should be 0.001-20 w/w%, preferably 0.1-5 w/w%.

Gymnema saponin enhances the whitening effects of the topical agents for dermatological use covered by the present patent as a result of synergism with 4-hydroxyphenyl-α-D-glucopyranoside. Gymnema saponin is a triterpenoid saponin obtained from extracts of gymnema inodrum or Gymnema sylvestre R. Br. Its salts include alkali metal salts such as Na and K salts, ammonium salt, basic amino acid salts, alkanol amine salts, and esters. These extracts can be used as is.
The content in topical agents for dermatological use should be 0.001-20 w/w%, preferably 0.1-5 w/w%.

Beat saponin enhances the whitening effects of the topical agents for dermatological use covered by the present patent as a result of synergism with 4-hydroxyphenyl-α-D-glucopyranoside. Beat saponin is an oleanolic acid glycoside obtained from beat extracts. Its salts include alkali metal salts such as Na and K salts, ammonium salt, basic amino acid salts, alkanol amine salts, and esters. These extracts can be used as is.
The content in topical agents for dermatological use should be 0.001-20 w/w%, preferably 0.1-5 w/w%.

Ellagic acid-related compounds are added to improve stabili-ty of the topical agents for dermatological use covered by the present patent and can be shown by general formula 18. Alkali metal salts of ellagic acid-related compounds include Na and K salts.
The content in topical agents for dermatological use should be 0.001-30 w/w%, preferably 0.05-10 w/w%.

Chemistry 6

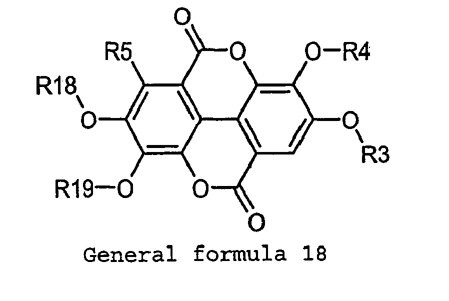

In general formula 18, R18, R19, R20 and R21 are hydrogen atoms, alkyl groups with 1 to 20 carbon atoms (such as methyl group, ethyl group and propyl group), acyl groups with 1 to 20 carbon atoms (such as acetyl group and propionyl group), polyoxy alkylene groups which can be shown by the formula - (CₘH₂ₘ-O)ₙH (in which m is 2 or 3, n is 1 or a greater integral number, prefer-ably a number between 4 and 50) (such as polyoxyethylene group and polyoxypropylene group), or sugar residues which are shown by general formula 19. R18, R19, R20 and R21 may be the same or different. R22 is a hydrogen atom, hydroxyl group, or alkoxy group with 1 to 8 carbon atoms.

Chemistry 7

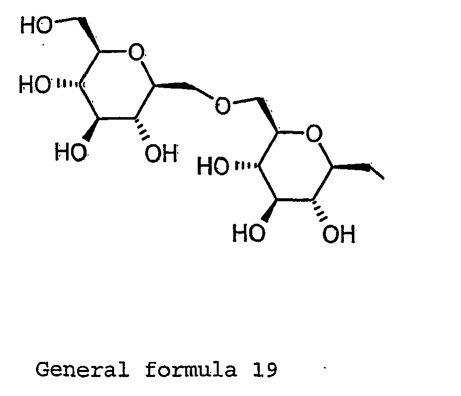

Examples of ellagic acid-related compounds and its alkali metal salts include ellagic acid, 3,4-di-O-methyl ellagic acid, 3,3'-di-O-methyl ellagic acid, 3,3',4-tri-O-methyl ellagic acid, 3,3',4,4'-tetra-O-methyl-5-methoxy ellagic acid, 3-O-ethyl-4-O-methyl-5-hydroxy ellagic acid and amritoside, as well as their alkali metal salts.
These ellagic acid-related compounds can be obtained from natural resources such as strawberry, Caesalupinia *spinosa,* eucalyptus, apple, *Coriaria japonica,* pinus radiata, bearberry, pomegranate, Phyllanthus *emblica L*., *Sapium sebiferum* leaf, Rhus chinensis leaf, Acacia catechu *L*., *Platycarya* strobilacea leaf, Terminalia chebula, *Camptotheca* acuminata, *Polygonum* bistorta *L., Lagerstroemia subcostata,* Sapium *discolor* root, *Sapium discolor* leaf, *Bischofia javanica, Lythrum salicaria L., Geranium* pratense *L., Euphorbia hirta L.,* Eucalyptus *citriodora, leaf, Euphorbia royleana, Psidium guajava fruit, Psidium guajava* cortex, *Mangife-ra indica L*., gall, *Syzygium* cumini fruit, *Syzygium cumini cor-tex, Phyllanthus emblica root, Phyllanthus emblica cortex, Phyl-lanthus emblica leaf, Agrimonia pilosa root, Psidium guajava leaf, Sapium* sebiferum *root cortex,* SHIDOKON (Kanppo name), CHINSYUSO (Kanppo name) and geranium herb.

Resorcinol derivatives show blood flow improving effects and cell activation effects. They inhibit the formation of melanin due to UV rays and promote the elimination of melanin. They also prevent the epidermis from drying, promote the skin metabolism, and prevent aging of the skin due to UV rays.
Specifically resorcinol derivatives include 4-n-ethyl resor-cinol, 4-n-butyl resorcinol, 4-n-hexyl resorcinol, and 4-isoamyl resorcinol.
The content in topical agents for dermatological use should be 0.0001-20 w/w%, preferably 0.01-10 w/w%.

Unless otherwise specified, all of the auxiliary agents listed above enhance the whitening effects of the topical agents for dermatological use under the present invention when concur-rently used with 4-hydroxyphenyl-α-D-glucopyranoside. Some of these auxiliary agents also increase the stability and/or safety of the topical agents.
None of these auxiliary agents affects 4-hydroxyphenyl-α-D-glucopyranoside in the topical agents when used with 4-hydroxy-phenyl-α-D-glucopyranoside in the indicated range of the con-tents. They remain stable for a long period of time and show excellent whitening effects. Their contents may be increased or decreased depending on the degree of expected effects. Each of these auxiliary agents can be used alone or in combination with one or more of the other agents.

4-hydroxyphenyl-α-D-glucopyranoside used in the present invention is explained below in great detail.
4-hydroxyphenyl-α-D-glucopyranoside is obtained as a result of α-binding of D-glucose with the phenol group of 1,4-dihydro-xybenzene. β-Arbutin, which is obtained as a result of β-binding of D-glucose with the phenol group of 1,4-dihydroxybenzene, is commonly used in topical agents for dermatological use because of its kin whitening effects. 4-Hydroxyphenyl-α-D-glucopyranoside is not only more effective than β-arbutin but is also more stable and safer when applied to the skin.

The content of 4-hydroxyphenyl-α-D-glucopyranoside in topical agents for dermatological use should be 0.01-30 w/w%, preferably 0.05-20 w/w% and more preferably 0.1-10 w/w%.

4-Hydroxyphenyl-α-D-glucopyranoside can be chemically manu-factured, but it can also be obtained by converting 1,4-dihydro-xybenzene into glycoside using enzymes deriving from bacteria. For example, this can be achieved using sucrose as a sugar donor and Leuconostoc *mesenterioides-*derived sucrose phosphorylase as an enzyme.
4-Hydroxyphenyl-α-D-glucopyranoside can also be obtained using soluble starch as a sugar donor and *Bacillus subtilis*-derived α-amylase as an enzyme. In this method, the use of amy-lase X-23 makes it possible to efficiently obtain 4-hydroxy-phenyl-α-D-glucopyranoside on an industrial scale.

How 4-hydroxyphenyl-α-D-glucopyranoside used in the present invention can be obtained using amylase X-23, a type of amylase obtained from Bacillus subtilis, is outlined below. Amylase X-23 decomposes glucan with α-1,4 bond in the presence of both phenol related compounds and glucan with α-1,4-bond, and transfers su-gar to the OH group of phenol related compounds by α-binding. The optimal pH for sugar transfer ranges between 5 and 8. Sugar transfer can be achieved in a stable manner at pH 5.5 and 30-70°C. Refer to Patent No. 2662667 Patent Gazette for greater detail.

Other ingredients commonly used for the production of topi-cal agents for dermatological use, including cosmetics and drugs, can be added, if necessary, to topical agents for dermatological use under the present invention. These ingredients include oil, antioxidants, surface active detergents, moisturizing agents, moistening agents, aromatics, water, alcohol, viscous agents, antiseptics, coloring agents, powder, drugs, chelating agents, and pH adjusting agents. These ingredients must be added in amounts which do not qualitatively or quantitatively affect the quality of topical agents for dermatological use covered by the present invention.

Topical agents for dermatological use covered by the present invention may be made available in any dosage form, including solutions such as toilet lotion, emulsified preparations such as milky liquid and cream, ointment, viscous gel, dispersion, and powder.

When manufacturing topical agents for dermatological use covered by the present invention in the form of lotion, emulsion and viscous gel, greater efficacy can be obtained by combining the following water-soluble viscous agents with lower alcohol such as ethanol and isopropanol: plant-derived macromolecules (such as gum arabic, tragacanth gum, galactan, Cyamoposis gum, carrageenan, pectin, quince seed (marmelo) extract and brown algae powder), microorganism-derived macromolecules (such as xanthan gum, dextran and pllulan), animal-derived macromolecules (such as collagen, casein, albumin and gelatin), starch (such as carboxymethyl starch and methylhydroxy starch), cellulose (such as methylcellulose, nitrocellulose, ethylcellulose, methylhydro-xypropylcellulose, hydroxyethylcellulose, cellulose ulfate, hydroxypropylcellulose, carboxymethylcellulose, crystalline cel-lulose, cellulose powder), vinyl macromolecules (polyvinyl alco-hol, polyvinylmethylether, polyvinylpyrolidone and carboxyvinyl-polymer), acryl macromolecules (such as polyacrylic acid and its salts and polyacrylimide), organic viscous agents (such as gly-cyrrhizic acid and alginic acid), and inorganic viscous agents (such as bentonite, hectolite, labonite, aluminum silicate mag-nesium and silicic anhydride).
The content of water-soluble viscous agents in topical agents for dermatological use should be 0.01-5 w/w%, preferably 0.1-3 w/w%, while the content of lower alcohol in topical agents for dermatological use should be 0.3-35 w/w%. It is desirable to adjust the ratio of 4-hydroxyphenyl-α-D-glucopyranoside and lower alcohol (weight) to 3:1 to 1:3.

### Working examples

4-Hydroxyphenyl-α-D-glucopyranoside used in all examples shown below is obtained by allowing amylase X-23 to act in the presence of 1,4-dihydroxybenzene and maltopentaose. All contents shown below are based on w/w%.

### Example 1: toilet lotion

A toilet lotion was prepared by the conventional method using the formula shown below.

| | |
|---|---|
| Propylene glycol | 5.0 |
| Ethanol | 14.0 |
| POE (20) oleyl ether | 0.5 |
| 4-Hydroxyphenyl-α-D-glucopyranoside | 0.5 |
| 2-Hydroxy-4-methoxybenzophenone-5-sodium sulfonate | 0.1 |
| Methylparaben | 0.1 |
| Citric acid | 0.01 |
| Sodium citrate | 0.1 |
| Chamomile extract | 2.0 |
| Water-soluble placenta extract | 2.0 |
| Sodium hyaluronate | 0.3 |
| Flavor | 0.05 |
| Ion exchanged water | Remainder |

### Example 2: emulsion

An emulsion was prepared by the conventional method using the formula shown below.

| | |
|---|---|
| Stearate | 3 |
| Cetyl alcohol | 2 |
| Petrolatum | 5 |
| Liquid paraffin | 10 |
| Polyoxyethylene (10) monooleate ester | 2 |
| Polyoxyethyleneglycol 1500 | 3 |
| Triethanolamine | 1 |
| 4-Hydroxyphenyl-α-D-glucopyranoside | 5 |
| Pantethine-S-sodium sulfonate | 10 |
| N'N-dimethyl PABA octyl ester | 5.0 |
| Hydroquinone monomethyl ether | 0.01 |
| Sodium hydrogen sulfite | 1.0 |
| Azelaic acid | 0.2 |
| Pyridoxine | 0.2 |
| Ion exchanged water | Remainder |
| Flavor | Q.S. |
| Antiseptic | Q.S. |

### Example 3: cream

A cream was prepared by the conventional method using the formula shown below.

| | |
|---|---|
| Propylene glycol | 5.0 |
| Yellow beeswax | 4.0 |
| Cetyl alcohol | 5.0 |
| Reduced lanolin | 5.0 |
| Squalene | 36.0 |
| Glyceryl monostearate | 2.0 |
| POE (20) sorbitan monolaurate | 2.0 |
| Methylparaben | 0.1 |
| Ethylparaben | 0.15 |
| 4-Hydroxyphenyl-α-D-glucopyranoside | 1.0 |
| Allantoin | 3.0 |
| Japanese angelica root extract | 0.2 |
| Crude sugar extract | 1.0 |
| Teprenone | 1.0 |
| Kojic acid | 1.0 |
| Flavor | 0.1 |
| Ion exchanged water | Remainder |

### Example 4: pack

A pack product was prepared by the conventional method using the formula shown below.

| | |
|---|---|
| Polyvinyl alcohol | 16.0 |
| Polyethylene glycol | 4.0 |
| Propylene glycol | 7.0 |
| Ethanol | 11.0 |
| Methylparaben | 0.1 |
| 4-Hydroxyphenyl-α-D-glucopyranoside | 7.0 |
| Dihydroxy aluminum allantoinate | 3.0 |
| Ascorbic acid | 1.0 |
| Nordihydroguaiaretic acid | 5.0 |
| Citric acid | 0.3 |
| Sodium citrate | 0.7 |
| Flavor | 0.1 |
| Ion exchanged water | Remainder |

### Example 5: scalp treatment (toilet lotion for scalp treatment)

A toilet lotion for scalp treatment was prepared by the conventional method using the formula shown below.

| | |
|---|---|
| 1,3 butylene glycol | 6.0 |
| Polyethylene glycol | 4.0 |
| Ethanol | 11.0 |
| POE (60) hydrogenated castor oil | 2.0 |
| Potassium hydroxide | 0.05 |
| Carboxyvinyl polymer | 0.2 |
| 2-Hexyldecylpalmitate | 11.0 |
| Squalene | 5.0 |
| Yellow beeswax | 0.5 |
| 4-Hydroxyphenyl-α-D-glucopyranoside | 10.0 |
| Allantoin | 4.0 |
| Antiseptic | 0.2 |
| Flavor | 0.1 |
| Ion exchanged water | Remainder |

### Example 6: ointment

Ointment was prepared by the conventional method using the formula shown below.

| | |
|---|---|
| Petrolatum | 40.0 |
| Stearyl alcohol | 15.0 |
| Japan wax | 15.0 |
| POE (10) oleate | 0.25 |
| Glyceryl monostearate | 0.25 |
| 4-Hydroxyphenyl-α-D-glucopyranoside | 6.0 |
| Allantoin | 1.0 |
| Sorbitol | 5.0 |
| Propylene glycol | 5.0 |
| Gentian extract | 0.3 |
| Ion exchanged water | Remainder |

### Example 7: powder

A powder was prepared by the conventional method using the formula shown below.

| | |
|---|---|
| Tranexamic acid | 0.1 |
| Calamine | 0.1 |
| Sulfur | 0.1 |
| oil soluble glycyrrhiza extract | 1.0 |
| Dextrin | 2.0 |
| Talc | 95 |
| Decaglycel stearate | 1.0 |
| 4-Hydroxyphenyl-α-D-glucopyranoside | 0.7 |

### Example 8: toilet oil

Toilet oil was prepared by the conventional method using the formula shown below.

| | |
|---|---|
| Tocopherol | 0.2 |
| 4-hydroxycinnamate | 0.2 |
| Allantoin | 0.5 |
| Ascorbyl palmitate | 0.2 |
| 4-Hydroxyphenyl-α-D-glucopyranoside | 1.0 |
| Retinol acetate | 0.3 |
| Evening primrose oil | 2.0 |
| Oil soluble glycyrrhiza extract | 1.0 |
| Squalene | Remainder |

All topical agents for dermatological use obtained in Examples 1 through 8 showed good skin whitening effects with only slight skin irritation and sensitization potential. They also showed good stability over time.

### Test 1

A clinical trial was conducted using a cream prepared accor-ding to the formula shown in Example 3 except that the content of 4-hydroxyphenyl-α-D-glucopyranoside was changed from 1.0 w/w% to 0.5 w/w% and a control cream prepared in accordance with the for-mula shown in Example 3 except that 4-hydroxyphenyl-α-D-gluco-pyranoside was replaced with β-arbutin.
A total of 12 volunteers (6 men and 6 women aged between 25 and 55 years) were enrolled. The cream covered by the present invention was applied to an area in the medial side of the right upper arm of 6 volunteers (3 men and 3 women). The β-arbutin-con-taining control cream was applied to the remaining volunteers. Application was made 3 times a day (every 8 hours) for 7 conse-cutive days. The application sites were exposed to UV rays of 1 MED (minimum erythema dose) after application 3 times a day star-ting the first day of application using a UVB light source. The volunteers were crossed over 30 days after completion of the first trial, and the trial was repeated in the same way at another site in the medial part of the right upper arm. The double-blind design was used.
The degree of skin blackening was compared 14 days after starting UV ray irradiation to assess the skin blackening preven-tion effects with the naked eye. The efficacy was rated using the following 5-degree scale: very effective, effective, slightly effective, ineffective and aggravated.
Results obtained are shown in Table 1.

**Table 1**

| | Control | Invented cream |
|---|---|---|
| Very effective | 1 (8.3%) | 3 (25.0%) |
| Effective | 3 (25.0%) | 6 (25.0%) |
| Slightly effective | 5 (41.0%) | 2 (16.7%) |
| Ineffective | 3 (25.0%) | 1 (8.3%) |
| Exacerbated | 0 | 0 |

The invented cream was more effective than the control cream (whose β-arbutin content was twice as large as that of 4-hydroxy-phenyl-α-D-glucopyranoside in the invented cream) in preventing skin blackening and no adverse reaction was observed. This fin-ding indicates that the invented cream is an excellent product.

### Effects of the invention

The topical agents for dermatological use covered by the present invention enhance the effects of 4-hydroxyphenyl-α-D-glucopyranoside due to synergistic effects because they combine 4-hydroxyphenyl-α-D-glucopyranoside, which is safe and stable and exerts good skin whitening effects even when used alone, and various auxiliary agents. Therefore, topical agents for dermatological use covered by the present invention show marked skin whitening effects and blackening prevention effects and effecti-vely prevent and relieve liver spots and freckles. They were also shown to be safe and stable and are extremely useful as cosmetics and therapeutic agents.

## Claims

1. A topical composition for dermatological use comprising 0.01 - 30 wt.% of 4-hydroxyphenyl-α-D-glucopyranoside, **characterised in that** it additionally contains a UV absorber in an amount of 0.01 - 10 wt.%.

2. A composition according to claim 1 wherein 4-hydroxyphenyl-α-D-glucopyranoside is present in an amount of 0.1 - 10 wt.%

3. A composition according to claim 1 or claim 2 wherein the UV absorber is present in an amount from 0.5 to 8 wt.%.

4. A composition according to any preceding claim wherein the UV absorber is selected from benzophenone UV absorbers and benzotriazole UV absorbers.

5. A composition according to any preceding claim wherein the UV absorber is selected from alkyloxy-benzophenones and alkylphenyl-benzotriazoles.

6. A composition according to any preceding claim wherein the UV absorber is selected from benzoate UV absorbents, anthranilate UV absorbents, salicylate UV absorbents, cinnamate UV absorbents, benzophenone UV absorbents, 3-(4'-methylbenzylidene)-d,l-camphor, 3-benzylidene- d,l-camphor, urocanic acid, ethyl urocanate, dibenzalazine, dianisoylmethane, 4-methoxy- 4'-t-butyldibenzoylmethane, and 5-(3,3-dimethyl-2- norbornylidene)-3-pentane-2-one.

7. A composition according to any preceding claim wherein the UV absorber is selected from 2-hydroxy-4-methoxybezophenone -5-sodium sulphonate, 2-(2'-hydroxy-5'-ethylphenyl)-benzotriazole, 2-(2'-hydroxy-5'-butylphenyl)-benzotriazole, and 2-(2'-hydroxy-5'-methylphenyl)-benzotriazole.

8. A composition according to any preceding claim further containing
| | |
|---|---|
| ethanol | 0.01 - 14 wt.% |
| propylene glycol | 0.01 - 11 wt.% |
| methylparaben | 0.01 - 10 wt.% |
| citric acid or salt thereof | 0.01 - 10 wt.% |
| chamomile extract | 0.01 - 10 wt.% |
| water-soluble placenta extract | 0.01 - 10 wt.% |
| sodium hyaluronate | 0.01 - 10 wt.% |
| poyvinyl alcohol | 0.01 - 16 wt.% |
| polyethylene glycol | 0.01 - 10 wt.% |
| nordihydroguaiaretic acid | 0.01 - 10 wt.% |
| tocopherol | 0.01 - 10 wt.% |
| 4-hydroxycinnamate | 0.01 - 10 wt.% |
| allantoin | 0.01-10 wt.% |
| retinol acetate | 0.01 - 10 wt.% |
| evening primrose oil | 0.01 - 10 wt.% |
| oil soluble glycyrrhiza extract | 0.01 - 10 wt.% |
the remainder being ion-exchanged water or cosmetic oil.

9. A composition according to any preceding claim further containing one or more of:
crude drugs and their extracts, hydroxycarboxylic acid and its salts, gentian extract, phenol derivatives and its salts, placenta extract, kojic acid and its derivatives, glucosamine and its derivatives, azelaic acid and its derivatives, pyridoxin and its derivatives, vitamin E-nicotinate, diisopropylamine-dichloroacetate, chitosan and its decomposition products, caffeic acid derivatives, bydroxycinnamate and its derivatives, Umbelliferae plant extracts, mycelial cultures and their extracts, plants leaves and their extracts, plant bark and its extracts,. hinokitiol, ginseng extract, sulfur, crude sugar extracts, molasses extracts, mucopolysaccharide, teprenone, retic acid, γ-pyrone glycoside, hydroxysalicylic acid glycoside, hydroxysalicylic acid fatty ester glycoside, biphenyl compounds, ceramides, substances with ceramide-like structures, pantothenic acid and its derivatives, sodium hydrogen sulfite, anti-inflammatory agents, and amino acids and their derivatives, aminoethyl compounds, alkylene diamine carboxylic acid derivatives, betaine derivatives, acyl methyl taurine, fibronectin, tyrosinase inhibitors, hederacoside and its salts, gymnema saponin, beet saponin and its salts, ellagic acid-related compounds and their alkaline metallic salts, and resorcinol derivatives.

10. A composition according to any preceding claim wherein the 4-hydroxyphenyl-α-D-glucopyranoside is obtained using α-amylase.

11. A composition according to any preceding claim wherein the α-amylase is amylase X-23.

12. Use of the combination of 4-hydroxyphenyl-α-D-glucopyranoside, and a UV absorber as defined in any preceding claim in the manufacture of a topical composition for dermatological use for lightening skin colour and/or preventing excessive darkening and/or preventing or reducing skin problems such as liver spots and freckles caused by injudicious exposure to ultraviolet irradiation.
